# EUROPEAN PATENT APPLICATION

(11) **EP 0 838 526 A1**
(43) Date of publication of application: **29.04.1998**
(21) Application number: 97306473.6
(22) Date of filing: 26.08.1997
(51) Int. Cl.: C12N 15/87, A61K 48/00

(54) **Human satellite microchromosomes as vectors for gene therapy**

(30) Priority: 26.10.1996 CN 96122757
(71) Applicant: Hunan Medical University, Changsha, Hunan (CN)
(72) Inventor: Xia, Jiahui, National Lab. of Medical Genetics, Changsha, Hunan (CN)
(74) Representative: Connell, Anthony Christopher

(57) **Abstract**

The present invention provides novel eukaryotic expression vectors which can accommodate large polynucleotides, such as polynucleotides encoding genes. The vectors of this invention can be stably maintained and autonomously replicated as extra chromosomal elements in eukaryotic cells, particularly, mammalian, and more particularly human, for use in somatic gene therapy.

It is another object of this invention to provide cell lines which can be used as sources of the novel expression vectors.

It is yet another object of this invention to provide a method for treating a disease or disorder caused by genetic deficiency or defect using the novel expression vector comprising a desired gene.

## Description

### BACKGROUND OF THE INVENTION

Gene therapy is one of the most promising methods for curing Mendelian genetic diseases. Already many experiments have been performed successfully to deliver genes to cells deficient in particular proteins, for example, hypoxanthine phosphoribosyl-transferase(Lesch-Nyhan syndrome), adenosine deaminase(severe combined immunodeficiency), purine nucleoside phosphorylase(severe combined immunodeficiency), glucocerebrosidase(Gaucher disease), alpha 1-antitrypsin (emphysema), growth hormone(short stature), low density lipoprotein receptor(familial hypercholesterolemia), phenylalanine pydroxylase(pehylketonuria), argininosuccinate synthetase(citrullinemia), beta-globin(thalassemia), factor IX(hemophilia), etc. for ultimate purpose of curing diseases resulting from deficient or defective genes.

In spite of the enormous potential for gene therapy, unfortunately, there has not been enough evidence for the clinical efficacy for gene therapy from about 106 clinical trials already conducted worldwide involving about 567 patients. Francis Collins, the director of the National Center for Human Genome Research(NCHGR), summed up the situation as:" None of the currently available techniques is clinically usefully for systemic gene delivery, the kind that can provide a permanent cure." (Marshall E. The trouble with vectors. Science 269:1052-1055(1995)).

To date clinical gene transfer has employed viral vectors either retrovirus vectors (about 72 percent of the protocols) or adenovirus vectors. There are several disadvantages associated with using recombinant viruses, for example, they can only accommodate several kilobases of DNA and they are difficult to control and ensure expression. Among other problems with these viral vectors are unstable transmission, low frequency of integration, immunogenicity and random insertional mutation.

Because of the large size of DNA in many genes from higher organisms, the size limitation and restriction of the retroviral vector are stifling. For example, Factor VIII gene in the human which encodes the blood-clotting factor deficient in hemophiliacs, spans at least 190kb(Gitschier, et al., Nature(London), 312:326, 1984). The gene that is defective in Duchenne's muscular dystrophy is estimated to include more than a million base pairs(1000kb). A striking feature of this gene is the protein-coding portion may be encoded by as little as 15kb of DNA(Monaco, et al. Nature(London), 302:575, 1983). Thus, there is a strong need for new vectors which would allow incorporation of a very large piece of polynucleotides, such as a gene, for use in gene therapy. Thus, it is the object of this invention to provide novel eukaryotic expression vectors for gene therapy which overcome many shortcomings of current gene therapy modality. The vectors of the present invention are derived from human satellite microchromosomes and can accommodate greater than 50 kilobase pairs of polynucleotides.

It is further object of the invention to use the instant vectors to make libraries containing large DNA sequences otherwise not possible with conventional vectors.

### SUMMARY OF THE INVENTION

The present invention provides novel eukaryotic expression vectors which can accommodate large polynucleotides, such as polynucleotides encoding genes. The vectors of this invention can be stably maintained and autonomously replicated as extra chromosomal elements in eukaryotic cells, particularly, mammalian, and more particularly human, for use in somatic gene therapy.

It is another object of this invention to provide cell lines which can be used as sources of the novel expression vectors.

It is yet another object of this invention to provide a method for treating a disease or disorder caused by genetic deficiency or defect using the novel expression vector comprising a desired gene.

### DETAILED DESCRIPTION

For the purpose of this invention, the phrase"expression vector" or "gene therapy vector" refers to a DNA vector capable of replication in selected mammalian host cells, particularly human, and expressing a desired protein for somatic gene therapy.

The satellite microchromosome is an extra microchromosome over the normal number of 46 human chromosomes. It exists in all of carriers' cells. The karyotype is 47, XX/XY+satellite microchromosomes. The satellite microchromosome has no harmful genetic effects and is transmitted in a stable manner during meiosis. There are numerous reports in the literature of supernumerary small chromosomes with or without satellites. Within this category is a subgroup consisting of harmless, familial extra, bisatellited microchromosomes. In such families, the extra satellite microchromosome is usually found incidentally and generally causes no adverse effects in the carrier. See in general: Journal of Heredity, P. N. Howard-Peebles, 70(5): 347-8(1979); Human Genetics, C. Ramos et al., 49:7-10(1979); Clinical Genetics, D.R.Romain, 16:183-190(1979); South African Medical Journal, A.H.Barnard and R.D.Smart, 58(12):485-8(1980); New England Medical Journal, D.R.Romain, 96(689):90-91(1981); and Steinbach P, et al., Hum Genet 1983; 65:155-164(1983).

Two families were identified by the inventor in the People's Republic of China carrying satellite microchromosome with normal phenotype during cytogenetic research. One family has 3 carriers with normal phenotype. Proband was a male, 30 years old, his sister 35 years old and his father 65 years old. Another family has 2 carriers with normal phenotype. Proband was a male, 28 years old and his daughter 2 years old. The blood and skin samples were collected from the probands of each family for chromosome analysis. The result showed that both peripheral blood lymphocytes and skin cells had this satellite microchromosome. The satellite microchromosome has no harm to human body and can transmit through generations steadily through gametes to somatic cells. It has telomere and centromere. These characteristics meet the requirements for the vectors for gene therapy. Thus, it is the intention of this application to provide isolated satellite microchromosome for use in gene therapy. It is further intention of this application to provide a method of treating diseases caused by deficient or defective genes by use of this satellite microchromosome as a novel vector via gene therapy.

### Construction of Cell Lines

Peripheral blood lymphocytes from the 2 probands were collected and EB virus was used to transform lymphocyte cell lines(Catalog No. of Chinese Mutational Cell Bank of National Lab of Medical Genetics: Lymphocyte CNLMG-B5537LC, Lymphocyte CNLMG-B5538LC). Skin samples from the 2 probands were also collected to make 2 fibroblast cell lines(Catalog No. of Chinese Mutational Cell Bank of National Lab of Medical Genetics: Fibroblast CNLMG-B5537SKIN, Fibroblast CNLMG-B5538SKIN). The four cell lines containing the satellite microchromosomes were deposited with the China Center for Type Culture Collection (CCTCC), Wuhan University, Wuhan, the People's Republic of China, on October 14, 1996, and assigned Deposit Nos. CCTCC C 96004 (for B5538SKIN), CCTCC C 96003 (for B5538LC), CCTCC C 96002 (for B5537SKIN), and CCTCC C 96001 (for B5537LC). The deposits were made under the terms of the Budapest Treaty on the international recognition of the deposit of micro-organisms for purposes of patent procedure.

### Characteristics of the Satellite Microchromosome

Chromosome G banding and N banding analysis showed the satellite microchromosome is composed of bisatellite and centromere.

Using the satellite microchromosome-specific probe pool constructed by microdissection, PCR as probe to do Fluorescent in Situ Hybridization (FISH) with carriers' metaphase peripheral lymphocytes, it was confirmed that the origin of the satellite microchromosome is the short arm part of group D (chromosome 13 to 15) and G (chromosome 21, 22) chromosomes. (H-X Deng, et al. Chromosome-band specific painting: chromosome in situ suppression hybridization using PCR products from a microdissected chromosome band as a probe pool. Hum Genet 1992; 89:13-17(1992)). The formation of the satellite microchromosome is due to the misdivision during the meiosis of gametes.

Research on molecular genetics verified that the short arms of human group D and G chromosomes are Nucleolus Organization Region (NOR) and the region for coding human ribosomal RNA (rRNA). This region has about 300 copies of human ribosomal genes. DNA of NOR takes part in the formation of nucleolus in the nucleus, and its transcription product-rRNA is the basal component of ribosomes where protein is synthesized. (J.E. Sylvester et al., The human ribosomal RNA genes: structure and organization of the complete repeating unit, Hum. Genet. 73:193-198(1986)). The copy number of rRNA gene in different person is not the same. Even in the different tissue cells of the same person or the different metabolic period of the same cell, the expression of rRNA gene is different, but it has no effect on the function of human body. So the variation of the short arms of group D and G chromosomes has no harmful genetic effect.

### Technical Route of This Invention

Although the methodology described herein contains sufficient detail to enable one skilled in the art to practice the present invention, a commercially available technical manual entitled MOLECULAR CLONING (Maniatis, et. al., Cold Spring Harbor Laboratory, Cold Spring Harbor, New York) may provide some additional details useful to assist practicing of some aspects of this invention. Accordingly, this manual is incorporated herein by reference.

Briefly, as an initial step, the satellite microchromosomes are isolated from the deposited cell lines. The techniques used for purifying the chromosome include sucrose density gradient, flow sorting, etc., which are standard in the art. Specific genetic function is conferred on this satellite microchromosome by ligating a polynucleotide, such as a gene, of interest operatively-linked, down stream from an appropriate promoter or control sequence. The appropriate DNA sequence may be inserted into the vector by a variety of procedures. In general, the DNA sequence is inserted into an appropriate restriction endonuclease site(s) by procedures known in the art. Preferably, the restriction endonuclease sites are those derived from infrequent cutters, such as NotI, BglI, etc. A person skilled in the art can readily create and/or detect such unique restriction sites and such procedures and others are deemed to be within the scope of those skilled in the art. If desired, the pieces after the restriction can be purified by pulsed-field gel electrophoresis.

The DNA to be inserted into the vector may comprise, in addition to the polynucleotide sequence coding the desired protein and a promoter, a suitable enhancer, and also any necessary ribosome binding sites, polyadenylation site, splice donor and acceptor sites, transcriptional termination sequences, and 5' flanking nontranscribed sequences. DNA sequences derived from t antigen SV40 splice site, and polyadenylation sites may be used to provide the required nontranscribed genetic elements.

As a further embodiment, for ease of manipulation and handling, the vector of the present invention can be modified to include one or more of the following:
a. a DNA or genomic DNA region encoding at least one selectable marker;
b. a DNA or genomic DNA region encoding a multiple cloning site; and
c. a promoter, enhancer, ribosome binding sites, polyadenylation site, splice donor and acceptor sites and transcriptional termination sequences.

The expression vector can also be modified to include an appropriate expression control sequence(s) (promoter) to direct mRNA synthesis. Suitable promoters which may be employed include, but are not limited to, the retroviral LTR, the SV40 promoter, and the human cytomegalovirus (CMV) promoter described in Miller et al., Biotechniques 7: 980-990 (1989), or any other promoter (e.g., cellular promoters such as eukaryotic cellular promoters including, but not limited to, the histone, RNA polymerase III, and β-actin promoters). Other viral promoters which may be employed include, but are not limited to, adenovirus promoters, thymidine kinase (TK) promoters, and B19 parvovirus promoters. The selection of a suitable promoter will be apparent to those skilled in the art from the teachings contained herein.

Transcription of the DNA encoding the desired polypeptides by higher eukaryotes is increased by inserting an enhancer sequence into the vector. Enhancers are cis-acting elements of DNA, usually about from 10 to 300 bp that act on a promoter to increase its transcription. Examples include the SV40 enhancer on the late side of the replication origin bp 100 to 270, a cytomegalovirus early promoter enhancer, the polyoma enhancer on the late side of the replication origin, and adenovirus enhancers.

The expression vector also can contain a ribosome binding site for translation initiation and a transcription terminator. The vector may also include appropriate sequences for amplifying expression.

The vector of the present invention could also be made to include a DNA region comprising a multiple cloning cassette sequence containing infrequent cutting by restriction enzymes to facilitate the insertion of a desired gene. Multiple cloning cassette sequence cartridges are commercially available from several different companies (Stratagene, Promega, New England Biolabs, etc.). A typical cassette sequence cartridge would include restriction sites for 8 - 11 different enzymes (i.e., EcoRI, SacI, SmaI, AvaI, NotI, BamHI, Xba I, Hinc II, Acc I, Sal I, Pst I, Hind III, etc.). The availability of these cassette cartridges are known to those skilled in the art.

Selectable markers, for mammalian cells, confer resistance to a specific selection agent once DNA conferring the resistance is transfected into individual cells possessing a genetic inheritance pattern appropriate for the selectable marker being used in the vector. There are a variety of different dominant and recessive selection agents known to those skilled in the art. Any one of the following genes and agents should be effective in terms of employing a selective system:
G418 resistance is selected by exposure to medium containing 100 to 800 ug/ml G418. G418 selects for cells deficient in the aminoglycoside phosphotransferease and are referred to as neomycin resistant cells. (Southern and Berg, J. Molec. Appl. Gen., 1:327-341, 1982; Colbere-Garapin et al., J. Molec. Biol., 150:1, 1981.)
HAT resistance for forward selection (converting a thymidine kinase minus cell to a thymidine kinase positive cell) is selected with complete medium supplemented with 100 uM hypoxanthine, 0.4 uM aminopterin, 16 uM thymidine and 3 uM glycine. HAT medium selects for variants defective in either hypoxanthine-guanine phosphoribosyl-transferase or thymidine kinase (Littlefield, Proc. Natl. Acad. Sci. USA, 50:568, 1963; Littlefield, Science. 145:709-710, 1964).(Littlefield, Proc. Natl. Acad. Sci. USA, 50:568, 1963; Littlefield, Science. 145: 709-710, 1964).
Hygromycin B resistance is selected by exposure to complete medium supplemented with 10-400 ug/ml hygromycin B. Hygromycin B selects for variants defective in the hytgromycin-B-phosphotransferase (Gritz and Davis, Gene, 25:179-188, 1983; Santerre, et al., Gene, 30:147, 1984; Palmer, et al., Proc. Natl. Acad. Sci. USA, 84:1055-1059, 1987).
Adenine phosphoribosyltransferase (APRT) positive variants are selected by exposure to medium supplemented with 25 uM alanosine, 50 uM azaserine and 100 uM adenine (Lowy, et al., Cell, 22:817, 1980; Adair, et al., Proc. Natl. Acad. Sci. USA, 86:4574-4578, 1989).
Xanthine-Guanine Phosphoribosyltransferase (SGPRT) positive variants are selected with complete medium supplemented with dialyzed fetal calf serum, 250 ug/ml xanthine, 15 ug/ml hypoxanthine, 10 ug/ml thymidine, 2ug/ml aminopterin, 25 ug/ml mycophenolic acid, and 150 ug/ml L-glutamine (Mulligan and Berg, Proc. Natl. Acad. Sci. USA, 78:2073-2076, 1981).
Methotrexate resistance is selected by exposure to complete medium supplemented with 0.01 uM-300 uM methotrexate and dialyzed fetal calf serum. Methotrexate selects for cells expressing high levels of dihydrofolate reductase (O'Hare, et al., Proc. Natl. Acad. Sci. USA, 78:1527, 1981; Simonsen and Levinson, Proc. Natl. Acad. Sci. USA, 80:2495-2499, 1983).
Deoxycoformycin resistant cells are selected by exposure to complete medium supplemented with 10 ug/ml thymidine, 15 ug/ml hypoxanthine, 4 uM 9-B-D-xylofuranosyl adenine (XylA), and 0.01-0.03 uM 2'deoxycoformycine (dCF). This selection for mutants expressed adenosine deaminase (ADS; Kaufman et al., Proc. Natl. Acad. Sci. USA, 83:3136-3140, 1986).

Introduction of the vector construct into the host cell can be effected by convention techniques used in the art such as calcium phosphate transfection, DEAE-Dextran mediated transfection, and microinjection (Davis, L., Dibner, M., Battey, I., Basic Methods in Molecular Biology, (1986)). Other techniques of transfection include: (1) encapsulation of insert-containing vectors in liposomes, (2) electroporation of vector into mitotic cell recipients to enhance its inclusion within the nucleus as cells progress into G1 phase of the cell cycle, and (3) injection of DNA-encoated particles into cells by employing a Biolistic Particle Delivery System (DuPont); this last procedure essentially shoots DNA-coated bullets into cells or tissues.

In an example of one embodiment, the gene or cDNA derivative of the gene that is defective in Duchenne's muscular dystrophy or myotonic dystrophy, or one of a number of other diseases associated with muscle dysfunction may be cloned into the satellite microchromosome vector. The chromosome is then introduced into cells or tissues ex vivo, or into animals by methods appropriate for the target as described below. The transfected chromosome is established as an integral component of the recipient cells where it is stably maintained and expressed. Recipient cells, tissues or animals that were initially dysfunctional because of a genetic defect they possessed are cured and become normal because of the expression and synthesis of the normal gene product introduced in the vector. When cells or tissues are transformed ex vivo, they are subsequently provided to a patient to be treated with the desired polypeptide. Examples of mammalian cells which may be transformed include, but are not limited to, embryonic stem cells, embryonic carcinoma cells, as well as hematopoietic stem cells, hepatocytes, fibroblasts, myoblasts, keratinocytes, endothelial cells, and bronchial epithelial cells.

## Claims

1. Cell line deposited with China Center for Type Culture Collection (CCTCC) on October 14, 1996 with CCTCC Designation CCTCC C 96004.

2. Cell line deposited with China Center for Type Culture Collection on October 14, 1996 with CCTCC Designation CCTCC C 96003.

3. Cell line deposited with China Center for Type Culture Collection on October 14, 1996 with CCTCC Designation CCTCC C 96002.

4. Cell line deposited with China Center for Type Culture Collection on October 14, 1996 with CCTCC Designation CCTCC C 96001.

5. Satellite microchromosome contained in the cell line of claim 1.

6. Satellite microchromosome contained in the cell line of claim 2.

7. Satellite microchromosome contained in the cell line of claim 3.

8. Satellite microchromosome contained in the cell line of claim 4

9. Satellite microchromosome of claim 5 comprising a polynucleotide for use in gene therapy.

10. Satellite microchromosome of claim 6 comprising a polynucleotide for use in gene therapy.

11. Satellite microchromosome of claim 7 comprising a polynucleotide for use in gene therapy.

12. Satellite microchromosome of claim 8 comprising a polynucleotide for use in gene therapy.

13. A method for gene therapy comprising transforming cells ex vivo that carry a deficient or defective gene with satellite microchromosme of claim 5 which contains the desired polynucleotide, and providing the cells transformed to a patient in need of such polynucleotide.

14. A method for gene therapy comprising transforming cells ex vivo that carry a deficient or defective gene with satellite microchromosme of claim 6 which contains the desired polynucleotide, and providing the cells transformed to a patient in need of such polynucleotide.

15. A method for gene therapy comprising transforming cells ex vivo that carry a deficient or defective gene with satellite microchromosme of claim 7 which contains the desired polynucleotide, and providing the cells transformed to a patient in need of such polynucleotide.

16. A method for gene therapy comprising transforming cells ex vivo that carry a deficient or defective gene with satellite microchromosme of claim 8 which contains the desired polynucleotide, and providing the cells transformed to a patient in need of such polynucleotide.
